# EUROPEAN PATENT APPLICATION

(11) **EP 2 336 927 A1**
(43) Date of publication of application: **22.06.2011**
(21) Application number: 11154813.7
(22) Date of filing: 12.03.2009
(51) Int. Cl.: G06F 19/00

(54) **Scientific image processing system**

(30) Priority: 18.09.2008 US 212699
(62) Divisional of application: 09154984.0
(71) Applicant: Nemaris LLC, New York, New York 10013 (US)
(72) Inventor: Schwab, Frank J., New York,, NY 10013 (US); Lafage, Virginie C., Brooklyn,, NY 11218 (US); Fath, Simone E., 94340, Joinville le Pont (FR); Schwab, Helmut, Princeton,, NJ 08540 (US)
(74) Representative: Kindermann, Peter

(57) **Abstract**

The present invention refers to a scientific image processing system comprising a first module (M1) for image acquisition of a plurality of first image formats and image conversion into a standardized second image format; and a second module (M2) executing specialized processes (33, 34, 35, 36) on the basis of said standardized second image format, wherein a third module (M3) controls supply logistics (OV; 46, 47) on the basis of the processing of said second module (M2), wherein said standardized second image format is kept in a first temporary storage area (41) and a second temporary storage area (42).

## Description

The present invention relates generally to a scientific image processing system and, particularly, to a modular image processing system embedded in a flexible hardware system both task and system adaptable to a wide variety of practical settings or needs in the sciences, with the software being stand-alone, available on an external portable storage medium such as a USB key or any other external portable memory device (EPM), consequently portable and not requiring computer installation as often negated within large institutional IP systems. The modules offering novel measurement and simulation options for specific applications in the sciences and medicine.

The proposed system may find equally useful application in medicine as also in biology, urban planning, or other sciences requiring the analysis and processing of images. In biology, the growth or formation of plant or animal specimens may be of interest, as may be development growth and alternative traffic connections in urban planning. The following presentation, for the purpose of illustration, concentrates on medical surgical applications, specifically in orthopedics, whether in the subspecialty of spine, of hip/knee, or other surgery areas. However, the system is equally applicable to other specialties and other sciences.

In the modern IP (Internet Protocol) environment, scientist of many specialties, from the medical professionals to biologists, and urban planners, often work with not only one, but with several computers, for example, one in the office, another in the laboratory, and the one at home. The work-related computers may be under central institutional control not permitting new software installation.

When images must be processed, they may arrive in a variety of formats, including, for example, not only the ".jpeg", ".png" and ".bmp" formats, but also the large variety of DICOM formats (Digital Imaging and Communications in Medicine), specifically for medical radiographs and MRI images (Magnetic Resonance Imaging). This image acquisition often requires much time, presenting a time-consuming nuisance to the practitioner, even the need for costly and time consuming re-taking of images in an acceptable format. The subsequently following image processing may require a variety of specialty-specific measurements or simulations of interventions, some not commonly available for image processing. Certain surgeries require specific supplies or implants from outside manufacturers. The simulation of these surgeries as possible with the proposed system can result in advance procurement and storage of supplies or implants for future need. Professional reports on the analysis of patient conditions and treatment recommendations must be written. Finally, the most active patient images must be stored in an easily transportable and accessible manner including the added measurements or intervention simulations. In the end, an archive must be available for the less active images accumulated over longer periods of time.

It is, therefore, an object of the present invention to render the work of the researcher or medical practitioner more efficient, specifically when working with images, for example in surgery, biology, and many other areas, by saving time and resources, and permitting optimal results by offering a task and system adaptive, portable, modular image processing system. Such time saving and result improvement may be important in competitive settings, but even more important for the benefit of medical patients.

According to the present invention, this object is achieved by the features defined in the present claim 1.

In detail, a sequence of functions may be conducted in various steps on different computers, requiring the intermediate storage and possibly the transport of images or prior research results from site to site, either physically on external storage media such as USB keys, or electronically. The necessary hardware modules for the above described software modules may be separate and available at different locations, requiring intermediate data storage or transport. These hardware modules may, however, also be more or less combined in larger and more complex PCs or in only one central processor.

Bits and pieces of the hereby offered adaptive software and hardware system, as well as large central computer based monolithic systems, may be available from various sources. However, the proposed composite, modular, and portable system and a large variety of proposed specific solutions to the problems for each of the indicated steps are novel as indicated in this patent application.

The further subclaims refer to further specific embodiments of the present invention.

For a more complete understanding of the present invention and the advantages thereof reference is now made to the following description taking in conjunction with the accompanying drawing, in which:
Figure 1 illustrates a simplified block diagram of the adaptive software and hardware system according to the present invention;
Figure 2 illustrates a simplified functional block diagram of the Image Acquisiton Module M1 of Figure 1;
Figure 3 illustrates a simplified functional block diagram of the Processing Module M2 of Figure 1; and
Figure 4 illustrates a simplified functional block diagram of the Supply Logistics Module M3 of Figure 1.

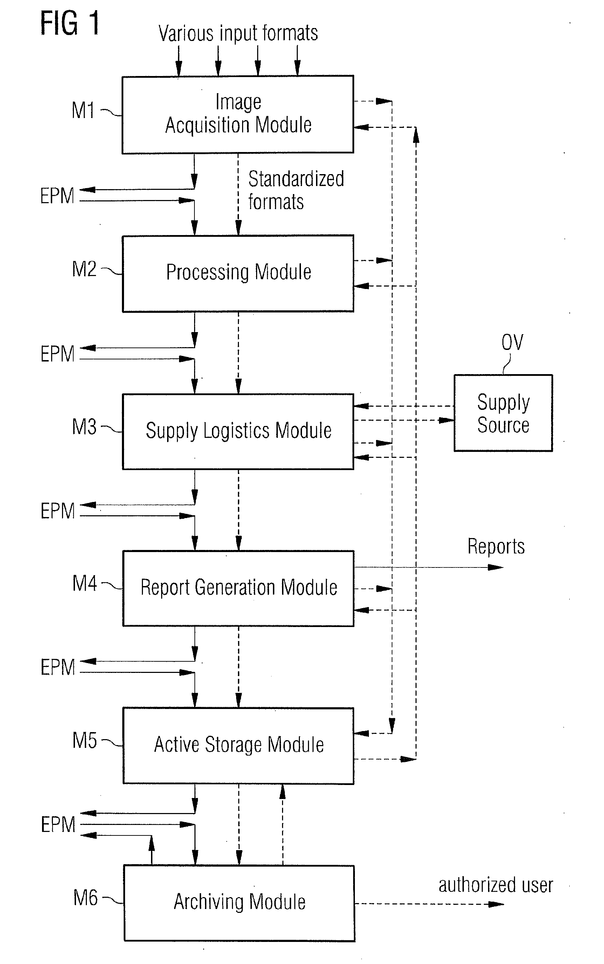

The software for the proposed adaptive software and hardware system as illustrated by Figure 1 may be supplied to the subscriber on an external portable memory EPM or storage medium such as a USB key, a CD, a wireless connected device, or any future external portable memory device, or, when so desired, can be downloaded from a specific website. The external portable storage or key-based option makes the software system independent, self-supporting, and easily transportable. It allows usage in environments, as in large hospitals or research laboratories with central computer systems, which otherwise are not accessible to new software due to restrictions imposed by administrator rights. More importantly, the modular hardware and software structure of the proposed system permits sequential utilization of the proposed system on a combination of different processors at different locations with data portability or transmission in between.

According to Figure 1 the activation of a first hardware module M1, i.e. the Image Acquisition Module, utilizes a first software module. This allows acceptance of transmitted or, in the case of medical practitioners, specifically surgeons, patient supplied images often presented on CDs in a variety of unique DICOM formats (Digital Imaging and Communications in Medicine), not readable by standard PCs. The still increasing large number of such DICOM variations can be addressed by periodic up-grades to the system. Similar situations with other image formats can be addressed in the same general manner.

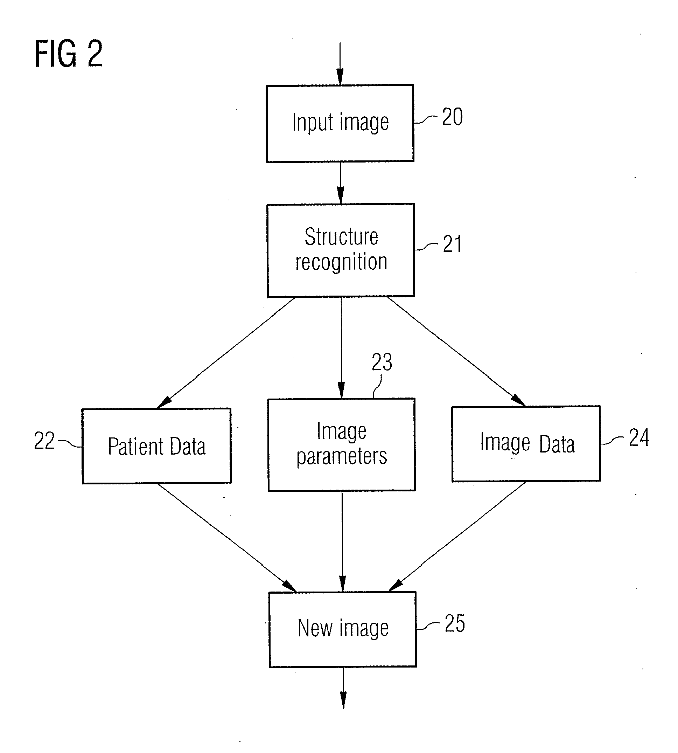

Figure 2 illustrates a simplified functional block diagram of the Image Acquisiton Module M1.

The functional block diagram and the following description of the same are of "typical" nature. Actual implementations may vary with the type of actual system application, availability of software or hardware, and future technology for image processing.

The incoming image dataflow is stored in an intermediate storage area 20. Subsequently, it is automatically fed to a "Structure Recognition" module 21. For example, so called "Notepad" software and other similar software types permit the analysis of a program inherent in data or image files, as commonly done by software architects to modify existing software programs. When opening a file in, for example, Notepad, typical segments or parts of code become apparent indicating what the meaning of the following detail program would be. For example the letters "<body ... " appear at the beginning of the central part of a data file. Subsequent insertion can result in the desired appearance of the file on screen. For example, appearance of *style="margin-left:150px;* ..." indicates a left side margin width on screen. Modification is easily possible. DICOM image files, for example, contain patient data, image format data, and other important data in specific segments of the image file. Other segments include the picture structure and the large volume of the actual image pixels. The "Structure Recognition" module 21 recognizes the different parts of, for example, a DICOM image (or images in other formats) and sends those separated to subsequent dedicated modules 22, 23 and 24 for patient data, image parameters, and actual image data. A final module 25 receives those portions and combines them into a new program of image file of standardized format for subsequent processing in the proposed system.

After image acquisition in the first module M1, the now standardized images can be kept in active memory, transmitted directly to a second module M2, i.e. Processing Module, see Figure 1, or stored on an external portable memory device EPM, such as a USB key, a CD or any other or future portable memory device for transport or transmission to the next module.

The second module M2, i.e. the Processing Module, often in a different location from the first module M1, i.e. Image Acquisition Module, is proposed in a wide variety of science specialty specific software implementations. The specifically selected processing module M2 allows a variety of the practitioner's specialty-specific measurements and, often more importantly, planning or intervention simulations. Many medical professionals in the environment of medical schools, and other scientist in their environment, do not only perform the functions of medical doctors or teachers, but also participate in research in laboratory settings and on- or off-site multicenter collaborative efforts. Therefore, the location of the second module M2 and the image processing may be in a different location and setting from the first module M1, i.e. the image acquisition. The proposed system offers specific innovative processing functions on a variety of processing modules for some scientific and medical specialties while others are still being developed and will be added by means of up-grades or new down-loads. For example, among the specialty processing functions for spine surgeons are simulations of osteotomies and other resections of bone or soft tissues.

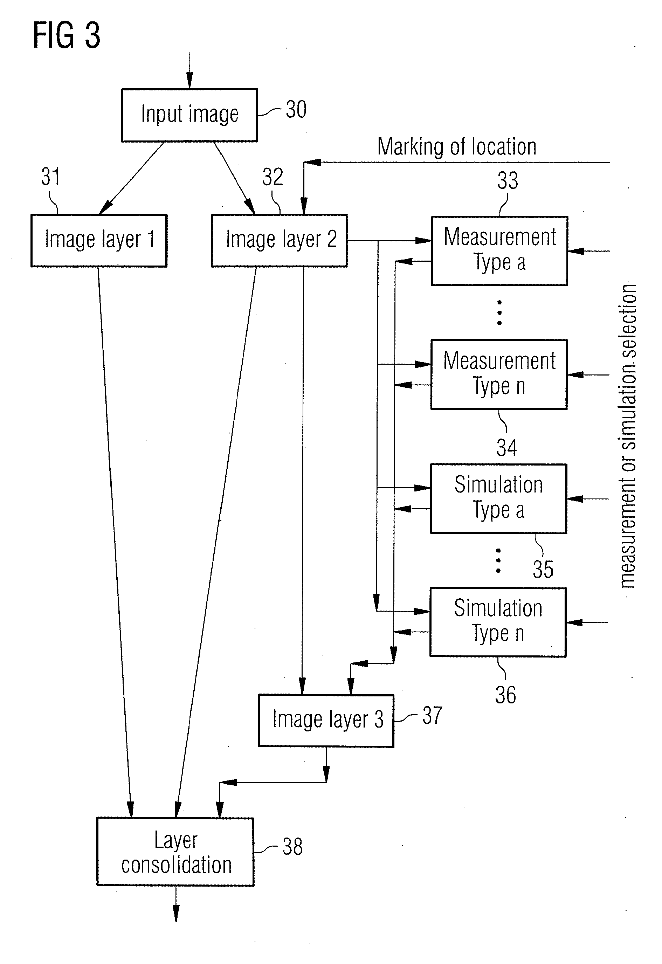

Figure 3 illustrates a simplified functional block diagram of the Processing Module M2.

The functional block diagram and the following description of the same are of "typical" nature. Actual implementations may vary with the type of actual system application, availability of software or hardware, and future technology for image processing. The incoming standardized image dataflow from Module M1 is stored in an intermediate image storage area 30. Subsequently, it is automatically kept in a first temporary storage area 31 called "Image Layer 1" and also fed to a second temporary storage area 32 called "Image Layer 2". It is a common approach to image processing (see, for example, the "Adobe Photoshop" software or others) to conserve the pixels of the original image in a separate file, as if in a separate layer of virtual film (file segment). Additions are usually done in, to say so, one or several separate layers or separate files of virtual film. At the end, after approval, the different virtual layers of film are consolidated into a single layer, resulting in the final image. In Module M2, operator input can mark the location of desired subsequent measurements or simulations by means of, for example, mouse movements and marking clicks. For a subsequent angle measurement, for example, two distinct lines are marked at whatever angle by means of their end-points. If marked in parallel, their distance may be measured. There may be several measurement options available in a system, from "Measurement Type a" 33 to "Measurement Type n" 34.

The markings may also refer to intended simulations of intended interventions, for example operations for separation, location changes, extractions, insertions, or other. There may be several simulation options available in a system, from "Simulation Type a" 35 to "Simulation Type n" 36. The position of the before mentioned markings, for example line locations and directions, are fed into the specialized processing units to execute the measurements and simulations, some of very sophisticated design. These operate only as selectively called upon and guided by the operator or user of the system. The results of the requested measurements are stored in quantitative data on third temporary storage area 37 called "Image Layer 3". The operator-performed simulations and consequent image changes are also stored on the third temporary storage area 37, i.e. "Image Layer 3". After review, the "Image Layer 1", "Image Layer 2" and/or "Image Layer 3" may be consolidated by a layer consolidation module 38 into the final image, now containing the original image, all the measurements and simulations, and their results. Operator comments can be added.

Planned and simulated medical interventions can result in variations in the relative locations of anatomic or specimen structures and, consequently, variations to measurements based upon landmarks or portions of anatomic or specimen structures. The proposed system allows the recalculation of various measurements performed on original imaging studies to reflect the expected impact of such simulated interventions. After image processing, the images containing the measurement and simulated intervention entries can be kept in a limited capacity active memory, transmitted directly to a third module M3, i.e. the Management of Supply Logistics Module, or stored on external portable memory EPM as shown in Figure 1, such as a USB key, a CD, or any other present or future portable memory device for physical transport or transmission to the third module M3.

In detail, the third module M3, for the Management of Supply Logistics, is also offered in a variety of application-specific implementations. Such a module may contain an inventory or catalogue of objects, supplies and implants offered and transmitted by preferred outside vendors OV or stored by the practitioner's institution. Selected items may be virtually moved into patient's images and implantation may be simulated. This can lead to the selection of desired supplies and implants needed for a specific planned intervention and, equally importantly, to their time-coordinated electronic ordering, and even their payment. Such electronic, internet based connection of intervention planning and supply logistics provides considerable work improvement, reducing valuable time and costs to user, customer and supplier alike.

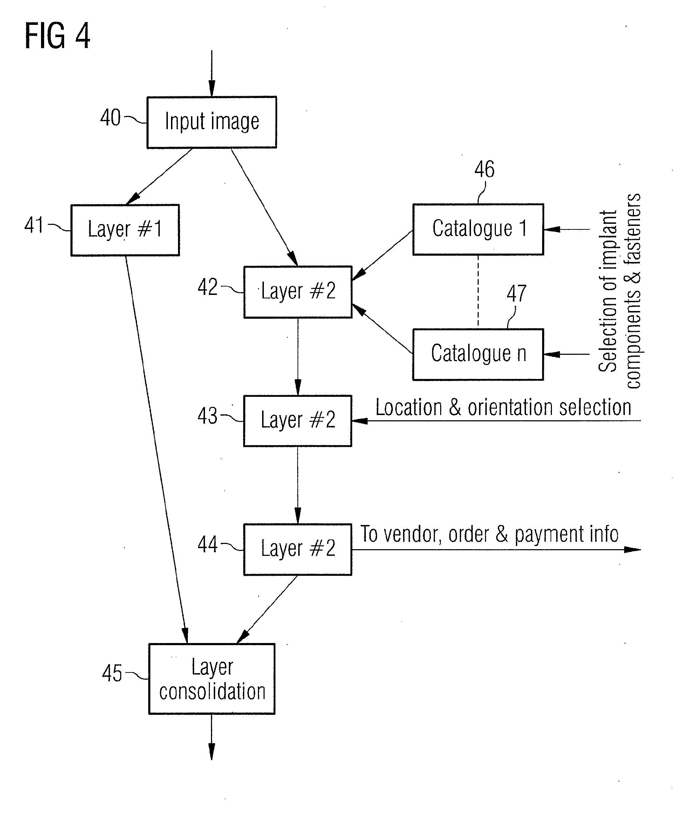

Figure 4 illustrates a simplified functional block diagram of the Supply Logistics Module M3.

The functional block diagram and its description in the following text are of "typical" nature. Actual implementations may vary with the type of actual system application, availability of software or hardware, and future technology for image processing. The incoming image directly from Module M1, or, if containing all subsequent processing information, as from Module M2, is stored in an intermediate image storage area 40 of Module M3. Subsequently, it is automatically kept in a first temporary storage area 41 called "Layer 1" and also fed to a second temporary storage area 42 called "Layer 2".

It is a common approach to image processing (see, for example, the "Adobe Photoshop" software or others) to conserve the pixels of the original image in a separate file, as if in a separate layer of virtual film (file segment). Additions are usually done in, to say so, one or several separate additional layers or separate files 43, 44, etc. of virtual film. At the end, after approval, the different virtual layers of film are consolidated by a consolidation module 45 into a single layer, resulting in the final image.

In Module M3, possibly a number of separate memory modules 46 to 47, named from possibly "Catalogue 1" to "Catalogue n", contain in electronic form the 3-D outlines, measurements, catalogue numbers, and also prices of all available parts, components, or existing image sections for possible incorporation into the image to be processed. The operator of the system selects from the various catalogues 46 to 47 the most suitable parts or components as well as fasteners, thereby transferring their stored images to the second temporary storage area 42, i.e. Layer 2. Subsequent separate operator input moves those parts or component images, including implants and their fasteners, now on Layer 2, to their desired location on Layer 2 and further adjusts their fit through rotation of those parts. This is a possible method in image processing as also used by other imaging software, for example, Adobe. Repetitive rejection and selection of new parts or fasteners can lead to an optimal solution of the existing operator (or surgeon) problem. When a final solution is found, the images remain in place, the catalogue part numbers are added, and the final selection decision is communicated back from Layer 2 to the vendor to initiate shipment for future use and also payment. Finally, the two layers 41 and 44 are consolidated by the consolidation module 45 into the final image, now containing the selected implants and fasteners in their proper place, as well as their listing.

After completion of this function, data can be transmitted directly to the next module, i.e. fourth module M4, or physically transported or transmitted there by means of intermediate storage on any external portable memory device EPM, as shown in Figure 1, such as an USB key, a CD, or any other present or future portable memory device.

The fourth module M4, i.e. the Report Generation Module, may be located in a different location from all the other modules M1 to M3, for example at the user's office or home. This module M4, see Figure 1, is also proposed in a variety of application-specific implementations. The module M4 provides ease of producing summary files or documents based on identification indicators often contained in the basic image information, specifically in DICOM-based images, and can combine this information with image files, measurements by the user and simulated intervention. The reports can be prepared assisted by a menu of preprogrammed routine report comments, on measurement values resulting from the processing modules, on individually provided information, and on the selection of recipient addresses or report storage locations from a given list. This report function can be programmed to generate reports in pre-established formats on standard forms.

After report generation, the images with the measurement and simulated intervention entries and attached report, can be kept in the local active memory of the fourth module M4, directly transmitted to the next module M5, i.e. the Active Storage Module, or be kept on external portable memory EPM, as shown in Figure 1, for example, on a USB key, a CD, or any other present or future portable memory device for transmission or transport to the next module or for sharing with other practitioners or researchers.

According to Figure 1, the fifth module M5, i.e. the Active Storage Module, may be any kind of portable memory device, nowadays preferably a USB key or CD, for easy transport and data independence from central systems. This may be the same memory device, USB key, CD, or any other kind of present or future portable memory device which contains the original software system. It may also be a different key, a CD writer, or, more importantly, a larger capacity back-up storage on the practitioner's office or home computer or a central computer, providing not only larger storage capacity, but also back-up protection against data or image loss. The proposed software system allows for the installation of an organized active image storage for ease of quick retrieval from various locations or for sharing with other practitioners.

According to Figure 1 the images may also be directly transferred to a sixth module M6, i.e. the Archiving Module.

The module M6, i.e. the Archiving Module, providing voluminous storage capacity for possibly years of images acquired and analyzed, is foreseen as being external, accessible through online connection possibly provided by one of the very large industrial data storage companies, but organized and under the control of the user of the proposed system.

Thus, a computer software system combined with a hardware system for scientific or medical image processing is disclosed being able to be of stand-alone application such as one that would have all of its basic program as well as most of the working data (images being investigated) embedded in external portable memory devices or mediums such as a USB keys, CDs, or any other present or future external portable memory devices and further consisting of a variety of adaptively selectable software and hardware modules for the specifically required, application specific processing functions with intermediate internal active storage or separate portable external memory or transmission capability as needed. In detail, the system comprises a software and hardware module for image acquisition or acceptance in practically all formats, including a large variety of DICOM formats, periodically upgraded to include newly appearing formats, a software and hardware module for executing specialized measurements and simulated interventions, a software and hardware module for supply logistics control, a software and hardware module for report generation, a software and hardware module for intermediate active storage, and a software and hardware module for archiving files containing image related information. Any combination of such modules combined in a few or only one central processor may be applicable according to the invention. Moreover, all indicated modules provided with intermediate internal storage or separate external portable memory as on USB keys, may have online or wireless transmission capability by e.g. an telecommunications network.

Although embodiments of the present invention and their advantages have been described in detail, it should be understood that various changes, substitutions and alterations can be made therein without departing from the spirit and scope of the invention as defined by the appended claims. For example, it will be readily understood by those skilled in the art that many of the features, functions, processes and methods described herein may be varied while remaining within the scope of the present invention. Moreover, the scope of the present application is not intended to be limited to the particular embodiments of the system, apparatus, method or steps described in the present invention. As one of ordinary skill in the art will readily appreciate from the disclosure of the present invention, systems, apparatuses, methods or steps presently existing or to be developed later, that perform substantially the same function or achieve substantially the same result as the corresponding embodiments described herein may be utilized according to the present invention. Accordingly, the appended claims are intended to include within their scope such systems, apparatuses, methods or steps.

## Claims

1. A scientific image processing system comprising:
a first module (M1) for image acquisition of a plurality of first image formats and image conversion into a standardized second image format; and
a second module (M2) executing specialized processes (33, 34, 35, 36) on the basis of said standardized second image format, **characterized by**
a third module (M3) controlling supply logistics (OV; 46, 47) on the basis of the processing of said second module (M2),
wherein
said standardized second image format is kept in a first temporary storage area (41) and a second temporary storage area (42).

2. The system according to claim 1, **characterized in that** the third module (M3) contains an inventory or catalogue (46, 47) of supplies and implants offered by outside vendors (OV) or stored by the practitioner's institution.

3. The system according to claim 2, **characterized in that** the third module (M3) selects from said inventory or catalogue suitable supplies.

4. The system according to claim 3, **characterized in that** the third module (M3) transfers suitable images of said selected supplies to said second temporary storage area (42).

5. The system according to claim 4, **characterized in that** the third module (M3) executes a final selection of suitable supplies and initiates shipment and payment of said finally selected suitable supplies.

6. The system according to any of claims 1 to 5, **characterized by**
a fourth module (M4) generating a report on the basis of said standardized second image format.

7. The system according to any of claims 1 to 6, **characterized by**
a fifth module (M5) storing intermediately said standardized second image format.

8. The system according to any of claims 1 to 7, **characterized by**
a sixth module (M6) archiving said standardized second image format.

9. The system according to any of claims 1 to 8, **characterized in that** said third module (M3) has online or wireless transmission capability.

10. The system according to any of claims 1 to 9, **characterized in that** it is embedded in an external portable memory device (EPM), thereby not requiring program installation on, for example, an institutional computer where installation and usage rights may be restricted.

11. The system according to any of claims 1 to 10, **characterized in that** said processes of said second module (M2) are measurements, medical or other scientific intervention simulations and planning, and the third module (M3) controls said supply logistics further on the basis of these measurements, medical or other scientific intervention simulations and planning of said second Module (M2).

12. The system according to claim 11, **characterized in that** the planned and simulated medical or other scientific interventions may result in variations in the relative locations of anatomic or specimen structures and, consequently, prior measurements applied by a user, where said system allows the automatic recalculation of various measurements performed on original imaging studies to reflect the impact of simulated or planned interventions.

13. The system according to claim 6, **characterized in that** the fourth module (M4) provides ease of report generation based on metadata or identification indicators contained in the original image information, specifically in DICOM-based images.

14. The system according to claim 8, **characterized in that** the sixth module (M6) provides voluminous and long-term storage capacity for possibly years of images acquired and/or analyzed.

15. The system according to any of claims 1 to 14, **characterized in that** at least one of the modules is at a different location than the other modules, being used at different times by the same or different operators, or where several of the modules are combined as, for example, in a home or institutional computer.
